(19) Europäisches Patentamt
European Patent Office
Office européen des brevets

(11) Numéro de publication: **0 231 714**
**A1**

(12) **DEMANDE DE BREVET EUROPEEN**

(21) Numéro de dépôt: **86420298.1**

(22) Date de dépôt: **11.12.86**

(51) Int. Cl.[4]: **A01N 43/04** , C07D 309/04 , C07D 307/10 , C07D 309/06 , C07D 335/02

La demande, qui était incomplète au moment du dépôt, est publiée telle quelle (article 93(2) CBE). Le passage de la description qui comporte manifestement une omission est présenté comme tel.

(30) Priorité: **16.12.85 FR 8518816**

(43) Date de publication de la demande: **12.08.87 Bulletin 87/33**

(84) Etats contractants désignés: **AT BE CH DE ES FR GB GR IT LI LU NL SE**

(71) Demandeur: **RHONE-POULENC AGROCHIMIE**
**14-20, rue Pierre Baizet**
**F-69009 Lyon(FR)**

(72) Inventeur: **Chene, Alain Les Terrasses de St Rambert**
**12, Chemin de Montpellas**
**F-69009 Lyon(FR)**
Inventeur: **Caruhel, Pascal**
**7 Rue Charles de Foucault**
**F-69330 Meyzieu(FR)**

(74) Mandataire: **Chrétien, François et al**
**RHONE-POULENC AGROCHIMIE BP 9163**
**F-69263 Lyon Cedex 09(FR)**

(54) **Compositions nématicides et insecticides à base de composés hétérocycliques.**

(57) Elles contiennent comme matière active un composé de formule :

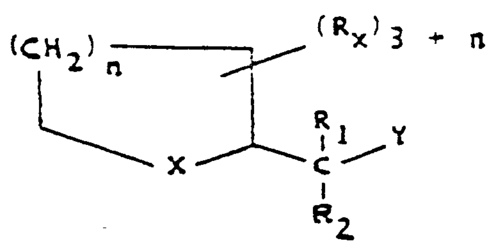

avec :
n = 1, 2 ou 3 ; X = O, S ;
$R_x$ = H, alcoyle $C_1$-$C_6$ éventuellement halogéné ;
$R_1$ et $R_2$ : identiques ou différents : H, halogène, alcoyle (halogéné) $C_1$ -$C_3$ ;
Y = H, XR, halogène, ammonium, sulfonium, phosphonium,
avec R = alcoyle (halogéné), C(X)R', $SO_2R'$, $-N=C(R_3R_4)$,
avec R' = alcoyle $C_1$-$C_6$, phényle éventuellement substitué ; $XR_3$, $NR_3R_4$ avec $R_3$, $R_4$ identiques ou différents : H, alcoyle $C_1$-$C_4$.
Application à la protection des plantes.

EP 0 231 714 A1

# COMPOSITIONS NEMATICIDES ET INSECTIDES A BASE DE COMPOSES HETEROCYCLIQUES

La présente invention concerne des compositions nématicides et insecticides, leur application pour la protection des plantes, et des dérivés hétérocycliques utilisables comme matières actives dans ces compositions.

Ces compositions sont caractérisées en ce qu'elles contiennent comme matière active, au moins un composé de formule :

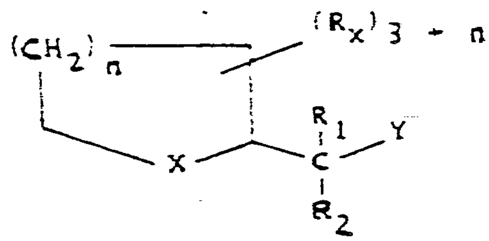

(I)

dans laquelle :

-n est un entier égal à 1, 2 ou 3,

-X est un atome d'oxygène ou de soufre

-$R_x$, identiques ou différents, sont des substituants choisis dans le groupe comprenant l'atome d'hydrogène ou un

radical alcoyle, éventuellement halogéné, de 1 à 6 atomes de carbone.

-$R_1$ et $R_2$, identiques ou différents, représentent un atome d'hydrogène, ou un atome d'halogène, ou un radical alcoyle ou alcoyle halogéné de 1 à 3 atomes de carbone.

-Y est

-un atome d'halogène, ou

-un cation ammonium, sulfonium ou phosphonium éventuellement substitué, ou

-un radical X R, dans lequel R est un alcoyle éventuellement halogéné, de 1 à 6 atomes de carbone, ou un radical C(X)-R′, $SO_2R'$ ou -N=C($R_3R_4$), dans lequel R′ est un alcoyle de 1 à 6 atomes de carbone, ou un groupe phényle éventuellement substitué par au moins un alcoyle de 1 à 4 atomes de carbone et/ou au moins un atome d'halogène,

ou encore un radical $XR_3$ ou

$NR_3R_4$, dans lesquels $R_3$ et $R_4$, identiques ou différents, peuvent être un atome d'hydrogène ou un alcoyle éventuellement substitué de 1 à 4 atomes de carbone ; ou, lorsque le composé présente un carbone asymétriqe, un de ses énantiomères ; ou, lorsque le composé présente plus d'un carbone asymétrique, un de ses diastéréiosomères, ou un de ses énantiomères correspondants.

Les composés qui présentent un carbone asymétrique existent selon les deux formes énantiomères R ou S. Dans ce cas, la formule donnée ci-dessus doit bien être comprise comme définissant aussi le mélange des deux en proportions, soit équivalentes (racémate), soit prépondérantes pour l'une ou l'autre d'entre elles.

Les composés qui présentent plus d'un carbone asymétrique existent selon des formes diastéréoisomères, chaque diastéréoisomère existant en général selon deux formes énantiomères. Dans ce cas, la formule donnée ci-dessus doit bien être comprise comme définissant aussi le mélange des diastéréoisomères et des énantiomères en proportions, soit équivalentes, soit prépondérantes pour l'une ou l'autre d'entre elles.

Des compositions préférées contiennent comme matière active au moins un composé de formule I dans laquelle :

-n est un entier égal à 1 ou 2,

-X est un atome d'oxygène,

-$R_x$, $R_1$ et $R_2$ sont des atomes d'hydrogène, ou des radicaux méthyle ou halogénométhyle et

-Y est un atome d'halogène.

L'invention a également pour objet des composés de formule :

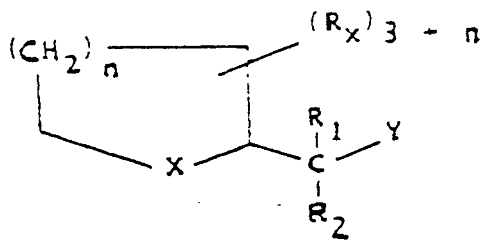

(II)

dans laquelle :

-n est un entier égal à 1, 2 ou 3,

-X est un atome d'oxygène ou de soufre

- $R_x$, identiques ou différents, sont des substituants choisis dans le groupe comprenant l'atome d'hydrogène ou un radical alcoyle, éventuellement halogéné, de 1 à 6 atomes de carbone.

-$R_1$ et $R_2$, identiques ou différents, représentent un atome d'hydrogène, ou un atome d'halogène, ou un radical alcoyle ou alcoyle halogéné de 1 à 3 atomes de carbone.

-Y est

-un atome d'halogène, ou

-un cation ammonium, sulfonium ou phosphonium éventuellement substitué ou

-un radical X R dans lequel R est un alcoyle, éventuellement halogéné, de 1 à 6 atomes de carbone, ou un radical CXR', $SO_2R'$ ou $-N=C(R_3R_4)$ dans lequel R' est un alcoyle de 1 à 6 atomes de carbone, ou un groupe phényle éventuellement substitué par au moins un alcoyle de 1 à 4 atomes de carbone et/ou au moins un atome d'halogène, ou encore un radical $XR_3$ ou $NR_3R_4$, dans lesquels $R_3$ et $R_4$, identiques ou différents, peuvent être un atome d'hydrogène ou un alcoyle éventuellement substitué de 1 à 4 atomes de carbone ;

-sous réserve que les substituants ci-dessus n'ont pas simultanément les définitions suivantes :

-n est un entier égal à 1 ou 2,

-X est un atome d'oxygène,

- $R_x$, $R_1$ et $R_2$ sont des atomes d'hydrogène, et -Y est un atome d'halogène.

ou lorsque le composé présente un carbone asymétrique, un de ses énantiomères ;

ou, lorsque le composé présente plus d'un atome asymétrique, un de ses diastéréiosomères, ou un de ses énantiomères correspondants.

Les composés qui présentent un carbone asymétrique existent selon les deux formes énantiomères R ou S. Dans ce cas, la formule donnée ci-dessus doit bien être comprise comme définissant aussi le mélange des deux en proportions, soit équivalentes (racémate), soit prépondérantes pour l'une ou l'autre d'entre elles.

Les composés qui présentent plus d'un carbone asymétrique existent selon des formes diastéréoisomères, chaque diastéréoisomère existant en général selon deux formes énantiomères. Dans ce cas, la formule donnée ci-dessus doit bien être comprise comme définissant aussi le mélange des diastéréoisomères et des énantiomères en proportions, soit équivalentes, soit prépondérantes pour l'une ou l'autre d'entre elles.

Certains composés de formule (I), dans laquelle Y représente un atome d'halogène, peuvent être préparés selon un procédé qui consiste à faire réagir un composé de formule :

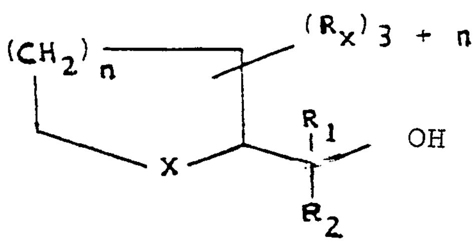

(III)

dans laquelle n, X, $R_x$, $R_1$ et $R_2$ ont la même signification que celle indiquée ci-dessus, avec un agent d'halogénation tel que $SOCl_2$, $P(O)Cl_3$, $PCl_3$, $PCl_5$, $PBr_3$, $P(O)Br_3$, en présence d'un accepteur d'acide tel qu'une amine tertiaire ou une pyridine, selon des méthodes en soi connues (voir notamment, entre autres références : J. MARCH, Advanced Organic Chemistry, 3rd Ed., Ed. John WILEY & SONS, 1985, p. 382 à 384).

Certains des composés de formule III sont connus. Les autres sont nouveaux et sont de formule III dans laquelle $R_1$, $R_2$, $R_x$, X et n ont la même signification que précédemment sous réserve que simultanément n ne soit pas égal à 1 ou 2, X ne soit pas l'oxygène ou le soufre et $R_1$, $R_2$, $R_x$ ne soient pas des atomes d'hydrogène ; les composés font également partie de l'invention.

Certains composés de formule (I), dans laquelle Y représente un atome d'halogène, peuvent être préparés selon un procédé qui consiste à faire réagir un composé de formule :

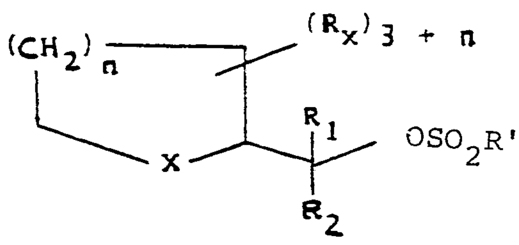

IV

dans laquelle n, X, $R_x$, $R_1$, $R_2$ et R' ont la même signification que celle indiquée ci-dessus, avec un sel de formule M-hal dans laquelle hal représente un atome d'halogène et M un atome de métal alcalin ou alcalinoterreux, notamment le lithium, le sodium, le potassium, selon des méthodes en soi connues (voir notamment la référence précédente, p. 382).

Les composés de formule IV sont nouveaux, à l'exception du tosylate de l'hydroxyméthyl-2 tétrahydropyranne, et font partie de l'invention. Ils peuvent être préparés selon un procédé, qui consiste à faire réagir un composé de formule III avec un dérivé de formule

$R'SO_2Cl$ V

dans laquelle R' a la même signification que celle indiquée ci-dessus, en présence d'un accepteur d'acide, tel qu'une base telle qu'une amine tertiaire ou une pyridine (voir notamment :

J. MARCH, Advanced Organic Chemistry, 3rd Ed., Ed. John WILEY & SONS, 1985, p. 357 à 358).

Certains composés de formule (I), dans laquelle Y représente un atome de fluor ou d'iode, peuvent également être préparés selon un procédé qui consiste à faire réagir un composé de formule :

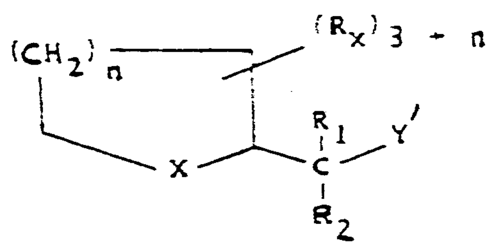

(VI)

dans laquelle n, X, $R_1$, $R_2$ et $R_x$ ont la même signification que celle indiquée ci-dessus, Y' représentant l'atome de brome ou de chlore, avec un sel de formule M-Y" (Y" représentant un atome de fluor ou d'iode et M ayant la même signification que celle indiquée ci-dessus), selon des méthodes en soi connues (voir notamment le référence précédente, p. 381).

Certains composés de formule (I), dans laquelle Y représente un atome d'halogène et X l'atome d'oxygène, peuvent également être préparés selon un procédé qui consiste à cycliser un alcool de formule :

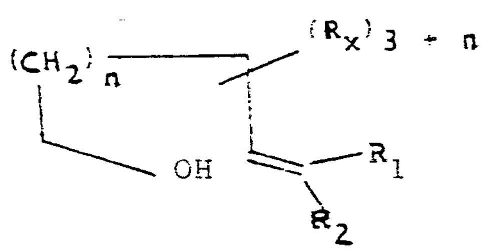

VII

dans laquelle n, $R_x$, $R_1$, $R_2$ ont la même signification que celle indiquée ci-dessus, selon des méthodes en

soi connues en présence d'un agent d'halogèno-éthérification, par exemple, à l'aide de N-bromosuccinimide ou d'hypobromite de tert-butyle (voir notamment M.L. MIHAILOVIC et coll., Bull. Soc. Chim. Beograd, 1982, 47 (8), 407-415), ou à l'aide du réactif tétracétate de plomb/M-hal (M et hal ayant la même signification que celle indiquée ci-dessus ; voir notamment S. MOTOHASHI et coll., Heterocyles, 1985, 23 - (8), 2035-2039).

La préparation de certains composés selon l'invention et les propriétés nématicides des compositions les contenant sont illustrées dans les exemples suivants, donnés à titre non limitatif.

Les structures des produits chimiques ont été vérifiées par spectrographie RMN (résonance magnétique nucléaire), et/ou IR (infra-rouge), et/ou SM (spectrographie de masse) et/ou microanalyse.

Exemple 1 : Préparation du bromométhyl-2 tétrahydropyranne (composé 1).

On charge, dans un ballon de 250 ml, 120 ml de toluène, 62 g de tribromure de phosphore, et 11,5 g de pyridine. On maintient la température en dessous de 0°C. On coule 80 g d'hydroxyméthyl-2 tétrahydropyranne et 4,6 g de pyridine. On maintient la température en dessous de 0°C durant l'addition des réactifs, sous agitation, et on poursuit l'agitation à la même température pendant 1 heure. On laisse revenir à la température ambiante puis reposer le mélange pendant une nuit. On filtre, lave au toluène et concentre par évaporation à sec. On obtient un résidu liquide jaune orangé que l'on distille sous le vide de la trompe à eau (18 mm Hg). On obtient 35,5 g d'un liquide incolore (Eb 74°C/18 mmHg) de bromométhyl-2 tétrahydropyranne, ayant pour formule :

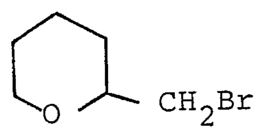

Exemple 2: Préparation du bromométhyl-2 tétrahydrofuranne (composé 2)

On opère selon un procédé analogue à celui de l'exemple 1, en remplaçant l'hydroxyméthyl-2 tétrahydro pyranne par l'hydroxyméthyl-2 tétrahydrofuranne. On obtient ainsi 60,0 g d'un liquide incolore - (Eb 70°C/18 mm Hg) de bromométhyl-2 tétrahydrofuranne, ayant pour formule :

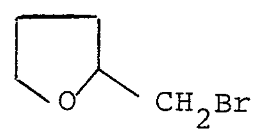

Exemple 3 : Préparation du bis bromométhyl-2,6 tétrahydropyranne (composé 3)

En opérant comme à l'exemple 1 à partir de 5,0 g de bis-hydroxyméthyl-2,6 -cistétrahydropyranne, 6,1 g de tribromure de phosphore, 1,3 g de pyridine et 30 ml de toluène, on obtient 0,7 g d'une poudre blanche, de point de fusion égale à 37°C de bis bromométhyl-2,6 -cistétrahydropyranne de formule :

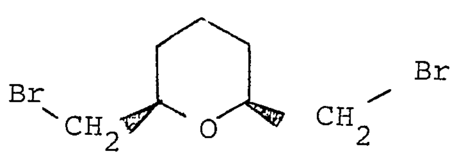

Exemple 1 : Préparation du (bromo-1-éthyl)-2 tétrahydropyranne (composé 4)

On opère selon un procédé analogue à celui de l'exemple 1, en remplaçant l'hydroxyméthyl-2 tétrahydropyranne par l'(hydroxy-1-éthyl)-2 tétrahydropyranne. On obtient ainsi 23 g d'un liquide incolore - ($n^{20}_{D}$ : 1,4855) de (bromo-1-éthyl)-2 tétrahydropyranne (mélange des deux diastéroisomères dans le rapport 55/45), ayant pour formule :

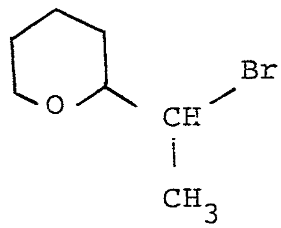

Exemple 5: Préparation du chlorométhyl-2 tétrahydropyranne (Composé 5)

On ajoute 22 cm3 de chlorure de thionyle à une solution de 30 g d'hydroxyméthyl-2 (tétrahydropyranne dans 44 cm3 de pyridine, de façon à ce que la température du milieu réactionnel se maintienne vers 40-45°C. Puis on chauffe, en poursuivant l'agitation, 8 heures à 45°C. L'huile résiduelle est reprise à l'éther et la phase éthérée est lavée à l'eau, puis avec une solution saturée de $NaHCO_3$, enfin avec une solution saturée de NaCl, avant d'être séchée sur $Na_2SO_4$ et concentrée. On obtient un résidu liquide que l'on distille sous un vide de 15 mm Hg. On obtient 20,2 g d'un liquide incolore (Eb 55°C/15 mm Hg) de chlorométhyl-2 tétrahydropyranne, ayant pour formule :

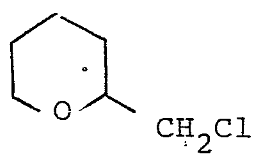

Exemple 6 : Préparation de l'iodométhyl-2 tétrahydropyranne (Composé 6)

On charge 5,4 g de bromométhyl-2 tétrahydropyranne (composé 1) et 4,5 g d'iodure de sodium dans 50 ml d'acétonitrile. On agite et on chauffe le mélange réactionnel 14 heures au reflux du solvant. On laisse refroidir à température ambiante, on filtre le précipité de bromure de sodium formé, puis on concentre par évaporation à sec. On obtient un résidu brun que l'on distille sous le vide de la trompe à eau (12 mm Hg). On obtient 3 g d'un liquide brun (Eb 88°C/12 mm Hg) d'iodométhyl-2 tétrahydropyranne, ayant pour formule :

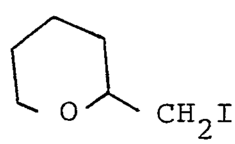

Exemple 7 : Préparation du tosyloxyméthyl-2 tétrahydropyranne (Composé 7)

On charge 35 g d'hydroxyméthyl-2 tétrahydropyranne dans 60 ml de pyridine. On agite et on refroidit le mélange réactionnel à 5°C. On additionne par portions 69 g de chlorure de tosyle, tout en maintenant la température en dessous de 10°C. En fin d'addition, on poursuit l'agitation 1 heure à 5°C, puis on laisse revenir à la température ambiante et maintient l'agitation pendant 2 heures. On laisse ensuite reposer le mélange pendant une nuit. On verse alors le mélange réactionnel sur de la glace, puis on extrait au toluène. La phase toluénique est lavée à l'HCl 10 %, puis à l'eau, avant d'être séchée sur $Na_2SO_4$ et concentrée. On obtient 33 g d'un solide blanc (F 73°C) de tosyloxyméthyl-2 tétrahydropyranne, ayant pour formule :

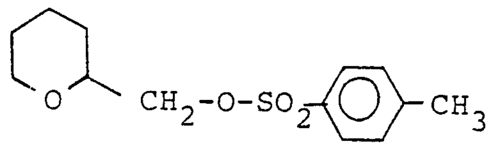

Exemple 8 : Préparation du fluorométhyl-2 tétrahydropyranne (Composé 8)

On charge 13,5 g de tosyloxyméthyl-2 tétrahydropyranne (composé 7) et 13,5 g de fluorure de potassium dans 60 ml d'éthylène glycol. On agite et on chauffe le mélange réactionnel 3 heures à 130°C. On laisse refroidir à température ambiante, puis on dilue le milieu avec 200 ml d'eau, puis on extrait avec deux fois 100 ml d'éther. Les extraits éthérés réunis sont lavés à l'eau, séchés sur $Na_2SO_4$ et concentrés par évaporation à sec. On obtient un résidu que l'on distille à la pression atmosphérique. On obtient 1,8 g d'un liquide incolore (Eb 110°C/760 mm Hg) de fluorométhyl-2 tétrahydropyranne, ayant pour formule :

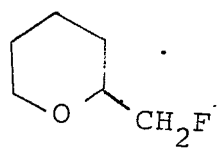

Exemple 9 : Préparation du mésyloxyméthyl-2 tétrahydropyranne (Composé 9)

On opère selon un procédé analogue à celui de l'exemple 7, en remplaçant le chlorure de tosyle par le chlorure de mésyle. On obtient ainsi 22 g d'une huile visqueuse jaune de mésyloxyméthyl-2 tétrahydropyranne, ayant pour formule :

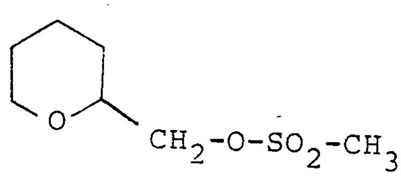

RMN $^{13}C$ à 62, 90 MHz ; $CDCl_3$ (ppm) : 75,1 / 72,4 /
TMS
68,3 / 37,5 / 27,3 / 25,5 / 22,8.

Exemple 10 : Préparation du diméthyl-6,6 iodométhyl-2 tétrahydropyranne (Composé 10)

A une solution de 44,3 g de tétracétate de plomb dans 200 ml de diméthoxy-1,2 éthane, on ajoute sous agitation, à 0°C, une solution de 7,2 g de méthyl-2 heptène-6 ol-2 dans 100 ml de diméthoxy-1,2 éthane. Puis on ajoute une solution de 15 g d'iodure de sodium dans 200 ml de diméthoxy-1,2 éthane, et l'agitation est maintenue durant 30 minutes à 0°C. Le mélange réactionnel est versé sur une solution de 1500 ml d'eau glacée et de 500 ml d'HCl 10 %, puis extrait à l'éther. La phase éthérée est lavée successivement avec une solution saturée de bicarbonate de sodium, avec une solution de thiosulfate de sodium à 10 %, et avec une solution saturée de chlorure de sodium, avant d'être séchée sur sulfate de sodium et concentrée par évaporation à sec. On obtient un résidu jaune que l'on distille sous un vide de 0,02 mm Hg. On obtient 4 g d'un liquide jaune (Eb 34°C/0,02 mm Hg ; $n_D^{20}$ = 1,5110) de diméthyl-6,6 iodométhyl-2 tétrahydropyranne, ayant pour formule :

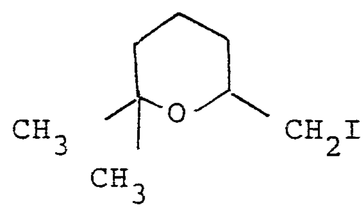

Exemple 11 : Préparation de l'(α, α'-diméthyl bromométhyl)-2 tétrahydropyranne (composé 11) et du diméthyl-5,5 bromométhyl-2 tétrahydropyranne (Composé 12)

On charge 12,8 g de méthyl-6 heptène-5 ol-1 et 19 g de N-bromosuccinimide dans 75 ml de tétrachlorure de carbone. On agite durant 8 heures à la température ambiante. Le mélange réactionnel est filtré et lavé avec une solution de $Na_2S_2O_3$ à 10 %, puis à l'eau, avant d'être séché sur $Na_2SO_4$ et concentré par évaporation à sec. On obtient. un résidu que l'on chromatographie sur colonne de silice (éluant heptane/acétate d'éthyle 98/2). On obtient 1,5 g d'un liquide incolore d'(α, α'-diméthyl bromométhyl)-2 tétrahydropyranne (composé 11) ayant pour formule :

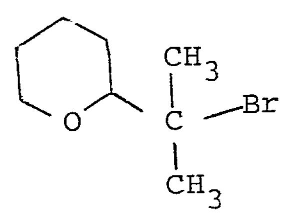

RMN $^{13}$C à 62,90 MHz : $\delta^{CDCl_3}_{TMS}$ (ppm) :

85,0/69,0/68,0/30,7/30,1/26,9/25,8/23,5.

En opérant de la même manière à partir de 14,2 g de diméthyl-2,2 hexène-5 ol-1 et 21 g de N-bromosuccinimide dans 150 g de tétrachlorure de carbone, on obtient 8,0 g de diméthyl-5,5 bromométhyl-2 tétrahydropyranne (composé 12), de point d'ébullition 48°C/0,05 mmHg, de formule :

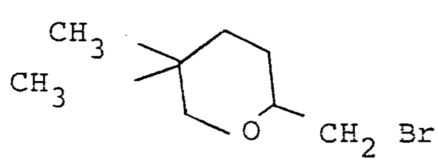

Exemple 12 : Préparation des deux diastéréoisomères du bromométhyl-2 méthyl-6 tétrahydropyranne : (composés 13 et 14)

On opère selon un procédé analogue à celui de l'exemple 11, en remplaçant le méthyl-6 heptène-5 ol-1 par l'heptène-6 ol-2. On obtient ainsi après chromatographie :

-5 g d'un liquide incolore du diastéréoisomère A,

-1 g d'un liquide incolore du diastéréoisomère B, du bromométhyl-2 méthyl-6 tétrahydropyranne, ayant pour formule :

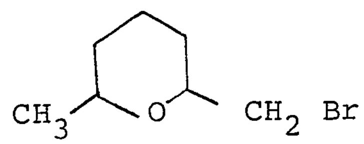

RMN $^{13}C$ à 62,90 MHz : $\delta_{TMS}^{CDCl_3}$ (ppm) :

- Isomère A : 77,1/74,6/35,6/33,0/29,5/23,5/21,6.
- Isomère B : 70,6/67,9/34,2/31,1/28,0/19,4/18,1.

Exemple 13 : Préparation des composés 15 à 36

En opérant selon un procédé analogue à celui de l'exemple 10 ou à celui de l'exemple 11, on obtient à partir des alcools correspondants, les composés 15 à 36 de formule :

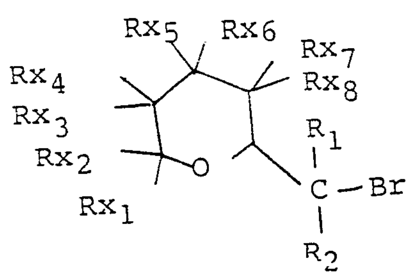

| n° | $R_1$ | $R_2$ | $Rx_1$ | $Rx_2$ | $Rx_3$ | $Rx_4$ | $Rx_5$ | $Rx_6$ | $Rx_7$ | $Rx_8$ |
|---|---|---|---|---|---|---|---|---|---|---|
| 15 | H | H | $CH_3$ | $CH_3$ | H | H | H | H | H | H |
| 16 | H | H | H | H | H | H | $CH_3$ | $CH_3$ | H | H |
| 17 | H | H | H | H | H | H | H | H | $CH_3$ | $CH_3$ |
| 18 | H | H | H | H | $CH_3$ | H | H | H | H | H |
| 19 | H | H | H | H | H | H | $CH_3$ | H | H | H |
| 20 | H | H | H | H | H | H | H | H | $CH_3$ | H |
| 21 | H | $CH_3$ | $CH_3$ | $CH_3$ | H | H | H | H | H | H |
| 22 | H | $CH_3$ | H | H | $CH_3$ | $CH_3$ | H | H | H | H |
| 23 | H | $CH_3$ | H | H | H | H | $CH_3$ | $CH_3$ | H | H |
| 24 | H | $CH_3$ | H | H | H | H | H | H | $CH_3$ | $CH_3$ |
| 25 | H | $CH_3$ | $CH_3$ | H | H | H | H | H | H | H |
| 26 | H | $CH_3$ | H | H | $CH_3$ | H | H | H | H | H |
| 27 | H | $CH_3$ | H | H | H | H | $CH_3$ | H | H | H |
| 28 | H | $CH_3$ | H | H | H | H | H | H | $CH_3$ | H |
| 29 | $CH_3$ | $CH_3$ | $CH_3$ | $CH_3$ | H | H | H | H | H | H |
| 30 | $CH_3$ | $CH_3$ | H | H | $CH_3$ | $CH_3$ | H | H | H | H |
| 31 | $CH_3$ | $CH_3$ | H | H | H | H | $CH_3$ | $CH_3$ | H | H |
| 32 | $CH_3$ | $CH_3$ | H | H | H | H | H | H | $CH_3$ | $CH_3$ |
| 33 | $CH_3$ | $CH_3$ | $CH_3$ | H | H | H | H | H | H | H |
| 34 | $CH_3$ | $CH_3$ | H | H | $CH_3$ | H | H | H | H | H |
| 35 | $CH_3$ | $CH_3$ | H | H | H | H | $CH_3$ | H | H | H |
| 36 | $CH_3$ | $CH_3$ | H | H | H | H | H | H | $CH_3$ | H |

Exemple 14 : Préparation du bromométhyl-2 tétrahydrothiopyranne (composé 37)

On opère selon un procédé analogue à celui de l'exemple 1, en remplaçant l'hydroxyméthyl-2 tétrahydropyranne par l'hydroxyméthyl-2 tétrahydrothiopyranne. On obtient ainsi 109 g d'un liquide incolore (Eb 58°C/0,5 mm Hg) de bromométhyl-2 tétrahydrothiopyranne, ayant pour formule :

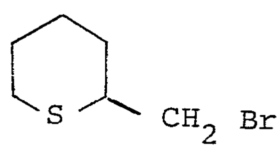

Exemple 15 : Teste nématicide in vitro (effet ovicide et larvicide)

Des racines de tomates, contaminées depuis environ deux mois et demi, sont lavées et broyées grossièrement. Les oeufs sont séparés des débris de racine par tamisage (maille 20 $\mu$). On prépare une suspension aqueuse renfermant environ 4000 oeufs embryonnés ou 4000 larves par ml d'eau distillée.

Le composé à tester est solubilisé dans l'acétone ou mis en suspension aqueuse à l'aide d'un broyeur de POTTER, dans de l'eau distillée additionnée de 0,02 % de monooléate de sorbitol oxyéthylé à 20 moles d'oxyde d'éthylène. Pour obtenir une gamme de concentrations, on effectue des dilutions successives à l'eau distillée.

Dans un tube à hémolyse, on mélange 1 ml de solution ou de suspension du composé à tester avec 1 ml d'une suspension d'oeufs ou de larves de Meloidogyne incognita. Après agitation manuelle, ce mélange est déposé sur des lames de microscope présentant chacune trois cuvettes. Chaque concentration fait l'objet de cinq répétitions.

Ces lames sont placées sur un support dans une boîte de Pétri, sur le fond de laquelle est déposé un papier filtre humide pour éviter la dessication du milieu pendant la durée de l'essai.

Cinq à huit jours après la mise en place de l'essai on examine chacune des lames à la loupe et on calcule le pourcentage de mortalité des larves de nématodes.

Dans ces conditions, on observe que les composés n°1 et 2, à la dose de 100 ppm, entraînent respectivement une mortalité totale des oeufs et de 50 % des larves et à la dose de 200 ppm, le composé 1 inhibe complètement l'éclosion des oeufs. A 10 ppm le composé 1 entraîne encore une mortalité totale de larves de Meloidogyne incognite.

Exemple 16 : Test nématicide en serre sur Meloidogyne incognita

A partir de racines de tomates contaminées, on obtient par tamisage comme à l'exemple précédent des oeufs du nématode étudié. On prépare une suspension aqueuse renfermant environ 1500 oeufs par ml d'eau distillée, dont au moins 10 à 15 % sont embryonnés.

On prépare un mélange, à parties égales, de sable de rivière, de terreau et de terre franche. Dans ce mélange, on incorpore par brassage la suspension d'oeufs de manière à avoir environ 3000 oeufs par litre de sol.

Le composé à tester est solubilisé :

-soit dans l'acétone puis mélangé avec de l'attaclay, le mélange étant, après homogénéisation, incorporé par brassage au sol contaminé,

-soit dans l'eau, auquel cas la solution est directement incorporée au sol contaminé.

Le sol contaminé et traité est réparti dans des pots (2 répétitions par dose) qui sont maintenus en enceinte humide ($\theta$ = 20°C et 100 % HR) pendant deux semaines.

Ensuite, on repique dans chaque pot deux plants de tomate (Lycopersicum esculentum) Variété Marmande, âgés d'environ un mois. Le sol est maintenu humide par arrosages réguliers.

Au bout de trois à quatre semaines, on arrache les plants et on lave leurs racines à l'eau.

L'efficacité nématicide est appréciée visuellement et notée en pourcentage de réduction du nombre de galles apparues sur les racines des plants ayant poussé sur un sol traité à celui des galles présentes sur les racines de plants repiqués dans un sol non traité, pris en comparaison.

Dans ces conditions, on observe que le composé 1 a une efficacité de 100 % à la dose de 10 kg/ha et les composés 2 à 6 une efficacité complète ( 90 %) à la dose de 50 kg/ha). Par ailleurs, on n'a pas constaté, à cette dose, de phytotoxicité.

Ces exemples illustrent clairement les excellentes propriétés nématicides des compositions selon l'invention. Elles sont donc appropriées pour être utilisées pour la lutte contre les nématodes parasites des plantes. Elles s'appliquent avantageusement à des doses de 0,5 à 50 kg de matière active à l'hectare de préférence de 1 à 25 kg/ha.

Pour leur emploi pratique, les composés selon l'invention sont rarement utilisés seuls. Le plus souvent ils font partie de compositions. Ces compositions, utilisables pour la protection des végétaux contre les nématodes et les insectes parasites contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment nématicides, insecticides ou fongicides) ou à propriétés favorisant la croissance des plantes -

(notamment des engrais) ou à propriétés régulatrices de la croissance des plantes. Plus généralement les composés selon l'invention peuvent être associés à tous les additifs solides ou liquides correspondant aux techniques habituelles de la mise en formulation. Ces doses d'emploi peuvent varier dans de larges limites, notamment selon la virulence de l'attaque et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

Selon ce qui a déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz

liquéfiés, etc...).

L'agent tensioactif peut être un agen émulsionnant, dispersant ou mouillant de type non ionique. On peut citer par exemple des se polyacryliques, des sels d'acides lignosulfon sels d'acides phénolsulfoniques ou naphtalène des polycondensats d'oxyde d'éthylène sur des ou sur des acides gras ou sur des amines gras phénols substitués (notamment des alkylphénol arylphénols), des sels d'esters d'acides sulf des dérivés de la taurine (notamment des alky des esters phosphoriques d'alcools ou de phén polyoxyéthylés. La présence d'au moins un age est généralement indispensable lorsque la mat et/ou le support inerte ne sont pas solubles que l'agent vecteur de l'application est l'ea

Les compositions utilisées dans l'in être sous des formes assez diverses, solides

Comme formes de compositions solides, les poudres pour poudrage. (à teneur en matiè pouvant aller jusqu'à 100 %).

Comme formes de compositions liquide à constituer des compositions liquides lors l'application, on peut citer les solutions, les concentrés solubles dans l'eau, les conc émulsionnables, les émulsions, les suspensio les aérosols, les poudres mouillables (ou po pulvériser), les granulés, les pâtes.

Les concentrés émulsionnables ou sol comprennent le plus souvent 10 à 80 % de mat les émulsions ou solutions prêtes à l'applic contenant, quant à elles, 0,001 à 20 % de ma En plus de la matière active et du solvant, émulsionnables peuvent contenir, quand c'est co-solvant approprié et de 2 à 20 % d'additi comme des stabilisants, des agents tensioact des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses selon l'invention :

Exemple A :

On prépare une suspension aqueuse comprenant :

- matière active (composé n° 1)................ 500 g/l
- agent mouillant (alcool synthétique en $C_{13}$ polyéthoxylé) ................................ 10 g/l
- agent dispersant (phosphate de polyaryl phénol éthoxylé salifié) ......................... 50 g/l
- antigel (propylèneglycol) .................. 100 g/l
- épaississant (polysaccharide) .............. 1,6 g/l
- biocide (hydroxyméthyl-4 benzoate sodé) .... 3,3 g/l
- eau .................................Q.S.P.1 litre.

On obtient ainsi une suspension concentrée fluide.

Exemple B -Suspension aqueuse :

On prépare une suspension aqueuse comprenant :

- matière active (composé n° 1)................ 100 g/l
- agent mouillant (alkylphénol polyéthoxylé .. 5 g/l
- agent dispersant (Naphtalène sulfonate de Na 10 g/l
- antigel (Propylèneglycol) .................. 100 g/l
- épaississant (Polysaccharide) ............. 3 g/l
- biocide (Formaldéhyde) .................... 1 g/l
- eau .................................Q.S.P.1 litre.

Exemple C -Suspension aqueuse :

On prépare une suspension aqueuse comprenant :

- matière active (composé n° 1)............... 250 g/l
- agent mouillant (alcool synthétique en $C_{13}$ polyéthoxylé ................................ 10 g/l
- agent dispersant (lignosulfonate de solium) 15 g/l
- antigel (urée) ............................ 50 g/l
- épaississant (Polysaccharide) ............. 2,5 g/l
- biocide (Formaldéhyde) .................... 1 g/l
- eau ...................................Q.S.P.1 litre.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple D, voici la composition de plusieurs poudres mouillables.

- matière active (composé n° 1 selon l'invention) 50 %
- alcool gras éthoxylé (agent mouillant)......... 2,5 %
- styrylphénol éthoxylé (agent dispersant)....... 5 %
- craie (support inerte) .......................42,5 %

Exemple E : Poudre mouillable à 10 %

- matière active (composé n° 1 selon l'invention) 10 %
- alcool synthétique oxo de type ramifié, en $C_{13}$ éthoxylé par 8 à 10 oxyde d'éthylène (agent mouillant) ....................................0,75 %
- lignosulfonate de calcium neutre (agent dispersant ........................................ 12 %
- carbonate de calcium (charge inerte) .......qsp 100 %

Exemple F : Poudre mouillable à 75 % contenant les mêmes ingrédients que dans l'exemple précédent, dans les proportions ci-après :

- matière active ............................ 75 %
- agent mouillant ..........................1,50 %
- agent dispersant ............................8 %
- carbonate de calcium (charge inerte) .......qsp 100 %

Exemple G : Poudre mouillable à 90 %

- matière active (composé n° 1 selon l'invention ..90 %
- alcool gras éthoxylé (agent mouillant ........... 4 %
- styrylphénol éthoxylé (agent dispersant) ........ 6 %

Exemple H : Poudre mouillable à 50 %

- matière active (composé n° 1 selon l'invention ..50 %
- mélange de tensio-actifs anioniques et non ioniques (agent mouillant) ......................2,5 %
- lignosulfonate de sodium neutre (agent dispersant) ........................................ 5 %
- argile kaolinique (support inerte) .............42,5 %

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale et de préférence organique. On a obtenu d'excellents résultats avec l'urée. Dans le cas d'une charge insoluble, celle-ci est de préférence minérale, comme par exemple le kaolin ou la bentonite. Elle est alors accompagnée d'agents tensio-actifs (à raison de 2 à 20 % en poids du granulé), adjuvants tensio-actifs dont plus de la moitié est constituée par au moins un agent dispersant, essentiellement anionique, tel qu'un poly(naphtalène sulfonate alcalin ou alcalino terreux) ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple I : Granulés dispersibles à 90 % de matière active

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple J : Granulés dispersibles à 75 % de matière active

Dans un mélangeur, on mélange les constituants suivants :

- matière active (composé n° 1) .................. 75 %
- agent mouillant (alkylnaphtalène sulfonate de sodium ........................................ 2 %
- agent dispersant (polynaphtalène sulfonate de sodium) ....................................... 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,16 et 0,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives; notamment fongicides, ces dernières étant sous la forme de poudres mouillables, ou de granulés ou suspensions aqueuses.

Les granulés pour épandage ont des dimensions comprises entre 0,1 et 2 mm et peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Voici deux exemples de composition de granulé :

Exemples K 1 et K 2 :

| | | |
|---|---|---|
| - matière active ........................ | 50 g | 200 g |
| - propylène glycol ...................... | 50 g | 50 g |
| - éther de cétyle et de polyglycol ...... | 2,5 g | 2,5 g |
| - polyéthylène glycol ................... | 35 g | 35 g |
| - kaolin (granulométrie : 0,3 à 0,8 mm) . | 910 g | 760 g |

Les composés selon l'invention peuvent encore être formulés sous forme de solutions organiques encapsulées, notamment par polymérisation interfaciale, dans des capsules à paroi polymériques, par exemple à base de polyamides de polyurées ou de polyamide urées. Ces capsules se trouvent à l'état de dispersion aqueuse concentrée que l'on peut diluer au moment de l'emploi pour obtenir une bouillie de pulvérisation.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

L'invention concerne de plus un procédé de traitement des végétaux contre les nématodes et insectes phytopathogènes.

Ce procédé est caractérisé en ce qu'il consiste à appliquer sur le lieu de culture de ces végétaux une quantité efficace d'une composition contenant comme matière active un composé selon la formule (I). Par "quantité efficace" on entend une quantité suffisante pour permettre le contrôle et la destruction des nématodes et insectes présents sur ces végétaux. Les doses d'utilisation peuvent toutefois varier dans de larges limites selon le parasite à combattre, le type de culture, les conditions climatiques, et selon le composé utilisé.

En pratique des doses allant de 1 g/hl à 500 g/hl correspondant sensiblement à des doses de matière active par hectare de 10 g/ha à 5000 g/ha environ donnent généralement de bons résultats.

**Revendications**

1) Compositions nématicides et insecticides pour la protection des plantes contre les nématodes et les insectes phytophages, caractérisées en ce qu'elles contiennent comme matière active, au moins un composé de formule :

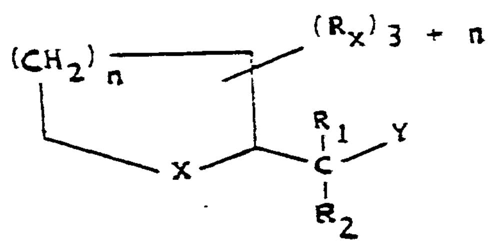

(I)

dans laquelle :

-n est un entier égal à 1, 2 ou 3,

-X est un atome d'oxygène ou de soufre

-$R_x$, identiques ou différents, sont des substituants choisis dans le groupe comprenant l'atome d'hydrogène ou un radical alcoyle, éventuellement halogéné, de l à 6 atomes de carbone.

-$R_1$ et $R_2$, identiques ou différents, représentent un atome d'hydrogène, ou un atome d'halogène, ou un radical alcoyle ou alcoyle halogéné de 1 à 3 atomes de carbone.

-Y est

-un atome d'halogène, ou

-un cation ammonium, sulfonium ou phosphonium éventuellement substitué, ou

-un radical X R dans lequel R est un alcoyle,

éventuellement halogéné, de 1 à 6 atomes de carbone, ou un radical C(X)-R', $SO_2R'$ ou $-N=C(R_3R_4)$ dans lequel R' est un alcoyle de 1 à 6 atomes de carbone, ou un groupe phényle éventuellement substitué par au moins un alcoyle de 1 à 4 atomes de carbone et/ou au moins un atome d'halogène,

-ou encore un radical $XR_3$ ou

$-N(R_3R_4)$, dans lesquels $R_3$ et $R_4$, identiques ou différents, peuvent être un atome d'hydrogène ou un alcoyle éventuellement substitué de 1 à 4 atomes de carbone ; ou, lorsque le composé présente un carbone asymétrique, un de ses énantiomères ; ou, lorsque le composé présente plus d'un carbone asymétrique, un de ses diastéréoisomères, ou un de ses énantiomères correspondants.

2) Compositions selon la revendication 1), caractérisées en ce que dans la formule I, n est un entier égal à 1 ou 2, X est un atome d'oxygène, $R_x$, $R_1$ et $R_2$ sont des atomes d'hydrogène ou des radicaux méthyle ou halogénométhyle et Y est un atome d'halogène.

3) Compositions selon la revendication 2, caractérisées en ce que la matière active est le bromométhyl-2 tétrahydropyranne.

4) Procédé de traitement des plantes pour leur protection contre les nématodes et insectes phytophages, caractérisé en ce que l'on applique, sur le lieu de culture, une composition selon l'une des revendications 1 à 3.

5) Nouveaux composés de formule :

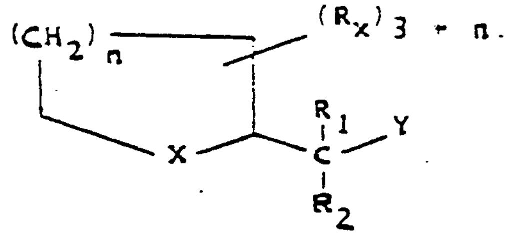

(II)

dans laquelle :
-n est un entier égal à 1, 2 ou 3,
-X est un atome d'oxygène ou de soufre
-$R_x$, identiques ou différents, sont des substituants choisis dans le groupe comprenant l'atome d'hydrogène ou un radical alcoyle, éventuellement halogéné, de 1 à 6 atomes de carbone.
-$R_1$ et $R_2$, identiques ou différents, représentent un atome d'hydrogène, ou un atome d'halogène, ou un radical alcoyle ou alcoyle halogéné de 1 à 3 atomes de carbone.
-Y est
-un atome d'halogène, ou
-un cation ammonium, sulfonium ou phosphonium éventuellement substitué,
-ou un radical X R dans lequel R est un alcoyle, éventuellement halogéné, de 1 à 6 atomes de carbone ou un radical C(X)-R', $SO_2R'$ ou -N = $C(R_3R_4)$ dans lequel R' est un alcoyle de 1 à 6 atomes de carbone, ou un groupe phényle éventuellement substitué par au moins un alcoyle de 1 à 4 atomes de carbone et/ou au moins un atome d'halogène, ou encore un radical $XR_3$ ou $N(R_3R_4)$, dans lesquels $R_3$ et $R_4$, identiques ou différents, peuvent être un atome d'hydrogène ou un alcoyle éventuellement substitué de 1 à 4 atomes de carbone ; ou, lorsque le composé présente un carbone asymétrique, un de ses énantiomères ; ou, lorsque le composé présente plus d'un carbone asymétrique, un de ses diastéréoisomères, ou un de ses énantiomères correspondants,
-sous réserve que les substituants ci-dessus n'ont pas simultanément les définitions suivantes :
-n est un entier égal à 1 ou 2,
-X est-un atome d'oxygène,
-$R_x$, $R_1$ et $R_2$ sont des atomes d'hydrogène, et -Y est un atome d'halogène.

6) Composés selon la revendication 5 dans la formule desquels
-n est égal à I ou 2,
-X est un atome d'oxygène,
-$R_1$, $R_2$ et $R_x$ identiques ou différents sont un atome d'hydrogène ou un radical alcoyle de 1 à 3 atomes de carbone éventuellement halogéné, au plus deux d'entre eux pouvant être simultanément un atome d'hydrogène,
-Y est un atome d'halogène.

7) Composés selon la revendication 6 dans la formule desquels Y est un atome de brome ou d'iode.

8) Procédé de préparation des composés selon la revendication 5 dans la formule desquels Y est un atome d'halogène, caractérisé en ce qu'on fait réagir un composé de formule :

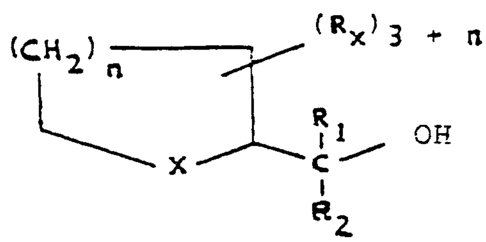

(III)

dans laquelle n, X, $R_1$, $R_2$, $R_x$ ont la même signification que dans la formule I, avec un agent d'halogénation tel que $SOCl_2$, $POCl_3$, $PCl_3$, $PBr_3$, en présence d'un accepteur d'acide.

9) Procédé de préparation des composés selon la revendication 5 dans la formule desquels Y est un radical $OSO_2R'$, caractérisé en ce qu'on fait réagir un composé de formule III avec un composé de formule $R'SO_2$ Cl (V), dans laquelle R' a la même signification qu'à la revendication 5, en présence d'un accepteur d'acide.

10) Procédé de préparation de composés selon la revendication 5 dans la formule desquels Y est Y" qui est un atome d'iode ou de fluor, caractérisé en ce qu'on fait réagir un composé de formule I, dans laquelle Y est Y' qui est un atome de chlore ou de brome, c'est-à-dire un composé de formule VI, avec un sel de formule M-Y" dans laquelle M est un cation de métal alcalin ou alcalinoterreux et Y" un atome d'iode ou de fluor.

11) Procédé de préparation de composés selon la revendication 5 dans la formule desquels Y est un atome d'halogène et X un atome d'oxygène, caractérisé en ce qu'on effectue la cyclisation d'un alcool de formule :

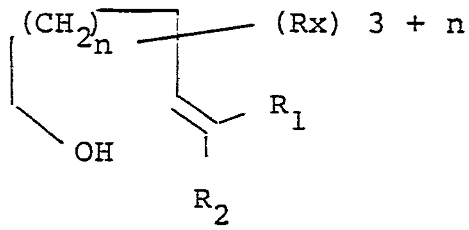

dans laquelle n, X, $R_1$, $R_2$, $R_x$ ont la même signification que dans la formule I, en présence d'un agent d'halogénation.

12) Procédé de préparation selon la revendication 11 caractérisé en ce que la cyclisation est effectuée en présence d'un agent d'halogènoéthérification tel qu'un dérivé bromé choisi dans le groupe comprenant le N bromosuccinimide et l'hypobromite de tertio-butyle en milieu solvant.

13) Procédé de préparation selon la revendication 11 caractérisé en ce que l'agent d'halogénoléthérification est un complexe tétracétate de plomb/Hal dans lequel M est un atome de métal alcalin ou alcalinoterreux et Hal est un atome d'halogène.

Revendications pour les Etats contractants suivants: AT, GR, ES

1) Compositions nématicides et insecticides pour la protection des plantes contre les nématodes et les insectes phytophages, caractérisées en ce qu'elles contiennent comme matière active, au moins un composé de formule :

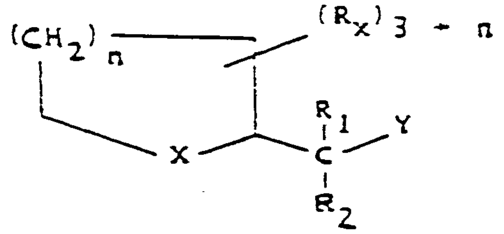

(I)

dans laquelle :

- n est un entier égal à 1, 2 ou 3,

-X est un atome d'oxygène ou de soufre

-$R_x$, identiques ou différents, sont des substituants choisis dans le groupe comprenant l'atome d'hydrogène ou un radical alcoyle, éventuellement halogéné, de l à 6 atomes de carbone.

-$R_1$ et $R_2$, identiques ou différents, représentent un atome d'hydrogène, ou un atome d'halogène, ou un radical alcoyle ou alcoyle halogéné de 1 à 3 atomes de carbone.

-Y est

-un atome d'halogène, ou

-un cation ammonium, sulfonium ou phosphonium éventuellement substitué, ou

-un radical X R dans lequel R est un alcoyle,

éventuellement halogéné, de 1 à 6 atomes de carbone, ou un radical C(X)-R', $SO_2R'$ ou $-N=C(R_3R_4)$ dans lequel R' est un alcoyle de 1 à 6 atomes de carbone, ou un groupe phényle éventuellement substitué par au moins un alcoyle de 1 à 4 atomes de carbone et/ou au moins un atome d'halogène,

-ou encore un radical $XR_3$ ou

$-N(R_3R_4)$, dans lesquels $R_3$ et $R_4$, identiques ou différents, peuvent être un atome d'hydrogène ou un alcoyle éventuellement substitué de 1 à 4 atomes de carbone ; ou, lorsque le composé présente un carbone

asymétrique, un de ses énantiomères ; ou, lorsque le composé présente plus d'un carbone asymétrique, un de ses diastéréoisomères, ou un de ses énantiomères correspondants.

2) Compositions selon la revendication 1), caractérisées en ce que dans la formule I, n est un entier égal à 1 ou 2, X est un atome d'oxygène, $R_x$, $R_1$ et $R_2$ sont des atomes d'hydrogène ou des radicaux méthyle ou halogénométhyle et Y est un atome d'halogène.

3) Compositions selon la revendication 2, caractérisées en ce que la matière active est le bromométhyl-2 tétrahydropyranne.

4) Procédé de traitement des plantes pour leur protection contre les nématodes et insectes phytophages, caractérisé en ce que l'on applique, sur le lieu de culture, une composition selon l'une des revendications 1 à 3.

5) Procédé de préparation des composés de formule :

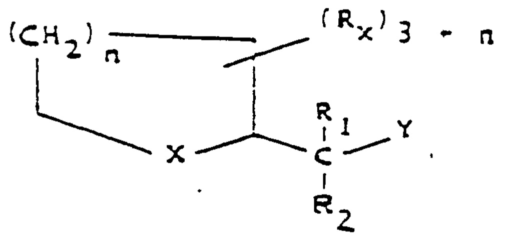

(II)

dans laquelle :

-n est un entier égal à 1, 2 ou 3,

-X est un atome d'oxygène ou de soufre

-$R_x$, identiques ou différents, sont des substituants choisis dans le groupe comprenant l'atome d'hydrogène ou un radical alcoyle, éventuellement halogéné, de 1 à 6 atomes de carbone.

-$R_1$ et $R_2$, identiques ou différents, représentent un atome d'hydrogène, ou un atome d'halogène, ou un radical alcoyle ou alcoyle halogéné de 1 à 3 atomes de carbone.

-Y est

-un atome d'halogène, ou

-un cation ammonium, sulfonium ou phosphonium éventuellement substitué,

-ou un radical X R dans lequel R est un alcoyle, éventuellement halogéné, de 1 à 6 atomes de carbone ou un radical C(X)-R′, $SO_2R'$ ou $-N=C(R_3R_4)$ dans lequel R′ est un alcoyle de 1 à 6 atomes de carbone, ou un groupe phényle éventuellement substitué par au moins un alcoyle de 1 à 4 atomes de carbone et/ou au moins un atome d'halogène, ou encore un radical $XR_3$ ou $N(R_3R_4)$, dans lesquels $R_3$ et $R_4$, identiques ou différents, peuvent être un atome d'hydrogène ou un alcoyle éventuellement substitué de 1 à 4 atomes de carbone ; ou, lorsque le composé présente un carbone asymétrique, un de ses énantiomères ; ou, lorsque le composé présente plus d'un carbone asymétrique, un de ses diastéréoisomères, ou un de ses énantiomères correspondants,

-sous réserve que les substituants ci-dessus n'ont pas simultanément les définitions suivantes :

-n est un entier égal à 1 ou 2,

-X est un atome d'oxygène,

- $R_x$, $R_1$ et $R_2$ sont des atomes d'hydrogène, et -Y est un atome d'halogène,

dans la formule desquels Y est un atome d'halogène,

caractérisé en ce qu'on fait réagir un composé de formule :

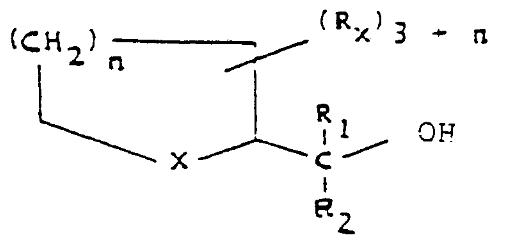

(III)

dans laquelle n, X, $R_1$, $R_2$, $R_x$ ont la même signification que dans la formule I, avec un agent d'halogénation tel que $SOCl_2$, $POCl_3$, $PCl_3$, $PBr_3$, en présence d'un accepteur d'acide.

6) Procédé de préparation des composés de formule selon la revendication 5 dans la formule desquels Y est un radical $OSO_2R'$, caractérisé en ce qu'on fait réagir un composé de formule III avec un composé de formule $R'SO_2$ Cl (V), dans laquelle R' a la même signification qu'à la revendication 5, en présence d'un accepteur d'acide.

7) Procédé de préparation de composés selon la revendication 5 dans la formule desquels Y est Y" qui est un atome d'iode ou de fluor, caractérisé en ce qu'on fait réagir un composé de formule I, dans laquelle Y est Y' qui est un atome de chlore ou de brome, c'est-à-dire un composé de formule VI, avec un sel de formule M-Y" dans laquelle M est un cation de métal alcalin ou alcalinoterreux et Y" un atome d'iode ou de fluor.

8) Procédé de préparation de composés selon la revendication 5 dans la formule desquels Y est un atome d'halogène et X un atome d'oxygène, caractérisé en ce qu'on effectue la cyclisation d'un alcool de formule :

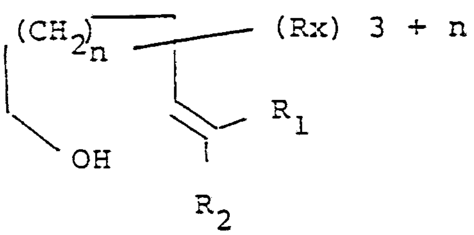

dans laquelle n, X, $R_1$, $R_2$, $R_x$ ont la même signification que dans la formule I, en présence d'un agent d'halogénation.

9) Procédé de préparation selon la revendication 8 caractérisé en ce que la cyclisation est effectuée en présence d'un agent d'halogènoéthérification tel qu'un dérivé bromé choisi dans le groupe comprenant le N bromosuccinimide et l'hypobromite de tertio-butyle en milieu solvant.

10) Procédé de préparation selon la revendication 8 caractérisé en ce que l'agent d'halogénoléthérification est un complexe tétracétate de plomb/Hal dans lequel M est un atome de métal alcalin ou alcalinoterreux et Hal est un atome d'halogène.

Office européen des brevets

# RAPPORT DE RECHERCHE EUROPEENNE

Numero de la demande

EP 86 42 0298

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | BULLETIN DE LA SOCIETE CHIMIQUE DE FRANCE, 1962, pages 177-182, Paris, FR; J. COLONGE et al.: "Etude sur les acools delta-éthyléniques" * Page 178, composés VI,VII,VIII,XI,XII * | 5-13 | A 01 N 43/04<br>C 07 D 309/04<br>C 07 D 307/10<br>C 07 D 309/06<br>C 07 D 335/02 |
| X | BULLETIN DE LA SOCIETE CHIMIQUE DE FRANCE, no. 7, 1966, pages 2320-2325, Paris, FR; L. GOUIN et al.: "Action des magnésiens du bromo-1 propyne-2 sur le chloro-2 tétrahydropyranne" * Page 2321, composé 14 * | 5-13 | |
| X | CHEMICAL ABSTRACTS, vol. 76, no. 25, 19 juin 1972, page 442, résumé no. 153486g, Columbus, Ohio, US; Yu.I. GEVAZA et al.: "Iodination of unsaturated epoxides. Reaction products", & UKR. KHIM. ZH. 1972, 38(3), 265-7 | 5-13 | |

--- -/-

| | |
|---|---|
| | DOMAINES TECHNIQUES RECHERCHES (Int. Cl.4) |
| | A 01 N 43/00<br>C 07 D 309/00<br>C 07 D 307/00<br>C 07 D 335/00 |

Le présent rapport de recherche a été établi pour toutes les revendications

| Lieu de la recherche | Date d'achèvement de la recherche | Examinateur |
|---|---|---|
| LA HAYE | 25-03-1987 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

# RAPPORT DE RECHERCHE EUROPEENNE

Numero de la demande

EP 86 42 0298

Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 70, no. 15, 14 avril 1969, page 378, résumé no. 68278x, Columbus, Ohio, US; V.V. SMIRNOV et al.: "Synthesis of 2-iodomethyl derivatives of 1,4-dioxane and tetrahydropyran", & KHIM. GETEROTSIKL. SOEDIN. 1968, (6), 1134 | 5-13 | |
| | --- | | |
| X | JOURNAL OF ORGANIC CHEMISTRY, vol. 48, no. 9, 6 mai 1983, pages 1557-1559, American Chemical Society, Easton, PA., US; H. NISHIYAMA et al.: "Silver-induced allylation of beta-bromo ethers with allylsilanes" * Page 1558, composés 1a,1e,1f * | 5-13 | |
| | --- | | DOMAINES TECHNIQUES RECHERCHES (Int. Cl.4) |
| X | CHEMICAL ABSTRACTS, vol. 59, no. 11, 25 novembre 1963, colonne no. 13290c, Columbus, Ohio, US; H.K. GOUCK et al.: "Compounds affecting the development of housefly larvae", & U.S. DEPT. AGR., ARS ARS 33-87, 8 pp.(1963) & CHEMICAL ABSTRACTS, 7TH COLLECTIVE SUBJECT INDEX, 1969, page 9708S: "Furan, 2-(chloromethyl)tetrahydro-," | 1-4 | |
| | --- | | |
| X | FR-A- 940 599 (J.R. GEIGY) * En entier * | 1-5 | |
| | ----- | | |

Le présent rapport de recherche a été établi pour toutes les revendications

| Lieu de la recherche | Date d'achèvement de la recherche | Examinateur |
|---|---|---|
| LA HAYE | 25-03-1987 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant